Europäisches Patentamt

European Patent Office

Office européen des brevets

(11) Veröffentlichungsnummer: **0 041 723**
**B1**

(12) **EUROPÄISCHE PATENTSCHRIFT**

(45) Veröffentlichungstag der Patentschrift: **18.09.85**

(51) Int. Cl.⁴: **C 12 N 1/18**

(21) Anmeldenummer: **81104393.4**

(22) Anmeldetag: **06.06.81**

(54) **Verfahren zur Entfernung der Bitterstoffe aus gebrauchter Bierhefe.**

(30) Priorität: **10.06.80 DE 3021653**

(43) Veröffentlichungstag der Anmeldung:
**16.12.81 Patentblatt 81/50**

(45) Bekanntmachung des Hinweises auf die
Patenterteilung:
**18.09.85 Patentblatt 85/38**

(84) Benannte Vertragsstaaten:
**AT BE CH DE FR GB IT LI LU NL SE**

(56) Entgegenhaltungen:
**DE-C- 215 417**
**DE-C- 744 920**
**FR-A-2 429 255**
**US-A-2 223 501**
**US-A-4 104 409**

**Die Akte enthält technische Angaben die nach
dem Eingang der Anmeldung eingereicht
wurden und die nicht in dieser Patentschrift
enthalten sind.**

(73) Patentinhaber: **Studiengesellschaft Kohle mbH
Kaiser-Wilhelm-Platz 1
D-4330 Mülheim/Ruhr (DE)**

(72) Erfinder: **Eisenbach, Wilhelm, Dr.
Lembkestrasse 6
D-4330 Mülheim/Ruhr (DE)**

(74) Vertreter: **von Kreisler, Alek, Dipl.-Chem. et al
Deichmannhaus am Hauptbahnhof
D-5000 Köln 1 (DE)**

Courier Press, Leamington Spa, England.

# Description

Die Erfindung betrifft ein Verfahren zur Entfernung der Bitterstoffe aus gebrauchter Bierhefe.

Gebrauchte Bierhefe wird nach Trocknen als Viehfutter verwendet. Diese Verwendung hat ihre Grenzen, da die Hefe einen Teil der aus dem Hopfen stammenden Bitterstoffe zu adsorbieren vermag, wodurch ein bitterer Geschmack der gebrauchten Bierhefe entsteht. Deshalb ist auch eine Verwendung der gebrauchten Bierhefe in anderen Bereichen, wie z.B. dem Humanbereich, nicht möglich.

Die Erfindung stellt sich die Aufgabe, ein Verfahren zu entwickeln, bei dem die Bitterstoffe der gebrauchten Bierhefe beseitigt werden können, um das Anwendungsgebiet für die gebrauchte Bierhefe zu erweitern.

Gelöst wird die Aufgabe dadurch, daß man die Bitterstoffe durch Behandeln einer wässrigen Suspension oder einer kolloidalen wässrigen Lösung der gebrauchten Bierhefe mit $CO_2$, Ethan, Ethylen und/oder Propan unter überkritischen Bedingungen des Drucks und der Temperatur in der fluiden Phase ohne Qualitätsverlust der Bierhefe entfernt.

Aus der DE—C—21 27 618 ist ein Verfahren zur Gewinnung von Hopfenextrakten durch Extraktion von lufttrockenem Hopfen mit Lösungsmittel bekannt, wobei man den Hopfen mit in Bezug auf Druck und Temperatur überkritischem $CO_2$, $SF_6$, $CHF_3$, $CHF_2Cl$, $CF_3Cl$, $CF_2=CH_2$, $C_3F_8$, $N_2O$, Äthan, Äthylen allein oder in Mischungen miteinander oder mit $CO_2$ extrahiert und aus der erhaltenen Lösung den Extrakt durch Senken des Druckes auf unterhalb des kritischen Druckes abscheidet. Mit diesem Verfahren können wahlweise gerbstoffreie oder gerbstoffhaltige Hopfenextrakte gewonnen werden.

Aus der DE—B—23 32 038 ist die Anwendung des Verfahrens zur Trennung von Fette und Öle enthaltenden Stoffgemischen, bei dem man die Fette bzw. Öle mit überkritischem $CO_2$ in Kontakt bringt, zum Desodorieren von Fetten und Ölen unter gegebenenfalls gleichzeitiger Verringerung der freien Fettsäuren darin bekannt, wobei man auf das zu reinigende Material $CO_2$ der Temperaturen von 150 bis 250°C und Drücken von 101,3 bis 253.3 bar einwirken lässt.

In der DE—C—215417 wird ein Verfahren zum Reinigen von Bierhefe beschrieben, in dem in Wasser befindliche Abfall-Bierhefe mit gasförmiger Kohlensäure behandelt und dadurch mechanisch gereinigt wird.

Die US—A—4 104 409 offenbart ein Verfahren zur Herstellung von Hopfenextrakten aus luftgetrocknetem Hopfen. Dabei wird getrockneter Hopfen in Kontakt mit überkritischen Lösungsmitteln gebracht und die Extrakte auf Normalbedingungen entspannt, wodurch Hopfenextrakte erhalten werden.

Diesem Stand der Technik kann der Durchschnittsfachmann die Lehre der vorliegenden Erfindung weder entnehmen noch ist sie ihm nahegelegt, zumal sich auch die Bitterstoffe in der gebrauchten Bierhefe gegenüber den Bitterstoffen im eingesetzten Hopfen oder eingesetzten Hopfenextrakt zumindest teilweise verändern und nach dem erfindungsgemässen Behandeln der gebrauchten Bierhefe mit überkritischem $CO_2$ und Entfernen der im $CO_2$ enthaltenen Bitterstoffe durch Behandeln mit einem Adsorptionsmittel eine andere Zusammensetzung der dabei gewonnenen Bitterstoffe vorliegt.

Durch die erfindungsgemässe Behandlung der gebrauchten Bierhefe ist es möglich, eine Bierhefe frei von Bitterstoffen und frei von bitterem Geschmack zu erhalten, die nicht nur ohne Probleme z.B. als Viehfutter, sondern auch für andere Zwecke, wie z.B. im Humanbereich, eingesetzt werden kann.

Außer $CO_2$ sind als Gase allgemein solche geeignet, deren kritische Temperatur unter 100°C liegt. Wichtig ist, daß keine gesundheitsbedenklichen Gründe oder ggfls. ökonomische Gründe gegen die Verwendung der Gase vorliegen. Derartige geeignete Gase sind insbesondere Äthan, Äthylen und Propan.

Verwendet werden die Gase im überkritischen Zustand, Dabei wird $CO_2$ im überkritischen Zustand besonders bevorzugt.

Bei dem Verfahren gemäss der Erfindung kann die gebrauchte Bierhefe als wässrige Bierhefesuspension oder als kolloidale Lösung der gebrauchten Bierhefe in Wasser bzw. eine Kombination beider, oder als getrocknete Bierhefe, z.B. sprühgetrocknete Bierhefe, eingesetzt werden.

Besonders bevorzugt wird die gebrauchte Bierhefe als wässrige Bierhefesuspension oder als kolloidale Lösung der gebrauchten Bierhefe in Wasser eingesetzt. Es ist besonders überraschend, daß man die Extraktion der Bitterstoffe aus einer wässrigen Bierhefesuspension bzw. einer kolloidalen Lösung der gebrauchten Bierhefe in Wasser vornehmen kann, da die einzige bisher bekannte Destraktion aus wässriger Phase die selektive Extraktion von Coffein aus einem Kaffee-Extrakt ist. Bei dieser Destraktion bleibt jedoch das Aroma in der wässrigen Phase, während im Falle der vorliegenden Erfindung die Bitterstoffe in das überkritische $CO_2$ übergehen.

Das erfindungsgemässe Verfahren wird bei Drucken durchgeführt, die von solchen über dem kritischen Druck bis zu 295 bar, vorzugsweise von 98 bar bis 200 bar reichen.

Als Temperaturen kommen solche über der kritischen Temperatur bis zo 10°C, vorzugsweise von 40 bis 70°C infrage.

Das Verfahren zur Behandlung der gebrauchten Bierhefe mit den überkritischen Gasen kann im Gegenstrom oder Gleichstrom durchgeführt werden. Derzeit bevorzugt wird die Durchführung des Verfahrens im Gleichstrom.

Bei einer weiteren bevorzugten Ausführung kann als zusätzliche Maßnahme das Verfahren im stationären Zustand durchgeführt werden. Das statische Mischverfahren, das z.B. zusätzlich zum Gleichstromverfahren durchgeführt werden kann, hat den Vorteil, daß man insbesondere die Hefeabscheidung auf Füllkörpern vermeiden kann, wie dies in den späteren Beispielen dargelegt wird.

Die zu verwendenden Gase wie z.B. das $CO_2$ werden während der gesamten Extraktion und Abscheidung unter überkritischen Bedingungen gehalten.

Das mit den Bitterstoffen beladene Gas, wie z.B. das überkritische $CO_2$ oder das überkritische Äthan, werden im Anschluß an die Beladung durch ein Adsorptionsmittel geleitet, um die Bitterstoffe zu entfernen. Das so gereinigte Gas kann anschließend im Kreislaufverfahren wiederverwendet werden.

Als Adsorptionsmittel wird vorzugsweise Aktivkohle verwendet. Alternativ ist es möglich, Molekularsiebe, Silicagel und/oder Aluminiumoxid als Adsorptionsmittel zu verwenden. Schließlich ist es auch möglich, das mit Bitterstoffen beladene überkritische Gas mit Wasser zu waschen und dadurch die Bitterstoffe zu entfernen.

Die nach der Entfernung der Bitterstoffe erhaltene gereinigte gebrauchte Bierhefe kann nach üblichen bekannten Techniken weiter aufgearbeitet werden. Beispielsweise kann die erfindungsgemäss gereinigte gebrauchte Bierhefe getrocknet werden, z.B. durch Sprühtrocknung.

Die Erfindung wird nachstehend anhand von Beispielen weiter erläutert.

### Beispiel 1

Durch ein mit VA-Füllkörpern (6 × 6 mm VA-Netzwendeln mit Mittelsteg) gefülltes VA-Rohr (Länge 5,5 m, lichte Weite 50 mm) wurde ein 10%ige Hefesuspension in Wasser von oben im Gegenstrom zu umlaufendem überkritischen $CO_2$ gepumpt. Bei einem Druck von 200 bar und einer Temperatur von 56°C wurden 250 ml/Std. zudosiert. Die ablaufende Bierhefe wurde aus einem Vorratsgefäß unterhalb des Füllkörperturms entspannt. Das überkritische $CO_2$ wurde mit einer Kreiselpumpe unter Reaktionsbedingungen mit ca 80 l/Std. anschließend durch einen mit A-Kohle (Korngröße 4—6 mm) gefüllten Reaktor umgepumpt; in der A-Kohle wurden die im $CO_2$ gelösten Bitterstoffe adsorbiert.

Die so behandelte Bierhefe schmeckte nicht mehr bitter. Ein gewisser Nachteil dieser Verfahrensweise ist, daß die Bierhefe leicht schäumt und mit dem $CO_2$ in die A-Kohle gelangt. Zudem kann die Gefahr bestehen, daß bei kleiner Strömungsgeschwindigkeit der Bierhefe sich der suspendierte Feststoff auf den Füllkörpern absetzen kann.

Durch eine geeignete Ausführungsform lässt sich durch Erhöhung der Durchsatzmenge dieser Nachteil der Ablagerung der suspendierten Bierhefe verweiden.

### Beispiel 2

In der unter Beispiel 1 beschriebenen Apparatur wurde die wässrige Bierhefesuspension im Gleichstrom mit dem überkritischen $CO_2$ von oben durch den Füllkörperturm gepumpt. Bei dieser Verfahrensweise gelangte keine Bierhefe mehr in die A-Kohle, das erhaltene Produkt schmeckte nicht mehr bitter.

### Beispiel 3

In der in Beispiel 1 beschriebenen Apparatur wurden wie in Beispiel 2 28 l einer 10 %igen wässrigen Bierhefesuspension bei einem Druck von 100 bar und einer Temperatur von 46°C von den Bitterstoffen befreit. Die eingepumpte Menge betrug 1 l/Std. bei einer umlaufenden $CO_2$-Menge von ca. 28 l/Std.

Die Menge der zudosierten Hefesuspension lässt sich auf das Vierfache steigern ohne Qualitätsverlust der entbitterten Hefe. Bei intensiverer Mischung von Bierhefe und $CO_2$ sind diesem Verfahren wesentlich weitere Grenzen gesetzt.

Die so entbitterte Flüssighefe wurde in einer konventionellen Apparatur sprühgetrocknet. Das erhaltene Pulver hatte das gleiche Aussehen wie das aus nicht behandelter Flüssighefe, es schmeckte nicht mehr bitter.

Die bei dem Versuch eingesetzte A-Kohle (600 g) wurde nach dem Versuch mit Chloroform extrahiert. Nach Abdampfen des Lösungsmittels konnte aus dem Extrakt ein dunkelbraunes, sehr bitter schmeckendes Öl erhalten werden. Die Menge betrug 12 g, entsprechend 2%, bezogen auf die eingesetzte A-Kohle oder ca 0,5% bezogen auf die in der Flüssighefe befindliche Trockensubstanz.

Die Gaschromatographie ergab folgendes Gaschromatogramm des aus der Aktivkohle rückgewonnenen Extraktes. Es zeigt sich, daß die Zusammensetzung des Extraktes eine andere ist, als die des ursprünglich dem Bier zugesetzten Hopfenextraktes.

| PEAK | MG | KOMMENTAR | | ANALYSE : A4341 |
|---|---|---|---|---|
| 1 | 92 | GLYCERIN | | EIS—OA—120—01 |
| 1A | 116 | HEXANSAEURE | 2,2 | W. EISENBACH |
| 1B | 106 | BENZALDEHYD ? (SEHR WENIG) | | |
| 2 | 122 | 2 - PHENYL - ETHANOL | 10,0 | MESSG : 7—Dec—79 |
| 3 | 144 | OCTANSAEURE | 14,7 | AUSW : 11—Dec—79 |
| 4 | 172 | OCTANSAEURE - ETHYLESTER | 4,2 F % | AUSWER : SCH |
| 5 | 172 | DECANSAEURE | 36,6 | |
| 6 | 200 | DECANSAEURE - ETHYLESTER | 21,0 | |
| 7 | 200 | DODECANSAEURE | 3,9 | |
| 8 | 228 | DODECANSAEURE - ETHYLESTER | 1,2 | |
| 9 | 214 | TETRADECANOL | 0,7 | |
| 10 | 242 | HEXADECANOL | 0,9 | |

0 041 723

MT

Peak    1        2    3   4            5   6            7   8        9                    10

### Beispiel 4

Anstelle des unter Beispiel 1 beschriebenen Füllkörperturms wurde ein statischer Mischer vom Typ Kenics-Mischer (Länge 600 mm, Durchmesser 10 mm) eingesetzt. Bei 175 bar und 47°C wurde Bierhefe und überkritisches $CO_2$ von unten durch diesen Mischer gepumpt. Bei einer eingepumpten Bierhefemenge von 250 ml/Std. und einer umlaufenden $CO_2$-Menge von 20 1/Std. wurde ein nicht mehr bitter schmeckendes Produkt erhalten.

Nach bisherigen Erfahrungen sollte das Verhältnis Bierhefesuspension zu $CO_2$ etwa 1:10 betragen, d.h. mit der gleichen $CO_2$-Menge lässt sich bei gröberer Strömungsgeschwindigkeit wesentlich mehr Bierhefe entbittern.

### Beispiel 5

Anstelle des in Beispiel 4 benutzten $CO_2$ wurde unter gleichen Reaktionsbedingungen Äthan im Kreislauf gefahren. Das erhaltene Produkt war von gleicher Qualität.

### Beispiel 6

In einer Apparatur, bestehend aus zwei Druckgefäßen von 1,8 und 5 l Inhalt, wurden in den kleineren 480 g A-Kohle (Korngröße 4—6 mm) und in den größeren 2700 g sprühgetrocknete Bierhefe eingefüllt. Mit Hilfe einer Kreiselpumpe wurde bei 200 bar und 55°C überkritisches $CO_2$ mit ca 80 1/Std. umgepumpt. Nach 24 Stunden wurde die Bierhefe ausgefüllt; sie schmeckte nicht mehr bitter und hatte ihr Aussehen nicht verändert.

Infolge der Feinheit des Hefepulvers ist eine relativ lange Reaktionszeit erforderlich, da die Durchmischung von Gas und Pulver nicht sehr günstig sein wird.

### Beispiel 7

In der unter Beispiel 6 beschriebenen Apparatur wurde $CO_2$ durch Äthan ersetzt. Unter gleichen Reaktionsbedingungen wurde nach 20 Stunden ein qualitativ gleiches Produkt erhalten.

Im allgemeinen ist zu den Mengenbereichen zu sagen, daß ein Mischungsverhältnis von $CO_2$ (Liter):Suspension und/oder kolloidale Lösung (Liter, 10—25%ig) von 5 bis 20:1 zu praktisch gut nutzbaren Ergebnissen führt. Es ist für den Durchschnittsfachmann ohne weiteres möglich, aufgrund einfacher Versuche für die jeweiligen Bedingungen das optimale Verhältnis herauszufinden.

### Patentansprüche

1. Verfahren zur Entfernung der Bitterstoffe aus gebrauchter Bierhefe in wässrigen Systemen, dadurch gekennzeichnet, daß man die Bitterstoffe durch Behandeln einer wässrigen Suspension und/oder einer kolloidalen wässrigen Lösung der gebrauchten Bierhefe oder von getrockneter Bierhefe mit $CO_2$, Ethan, Ethylen und/oder Propan unter überkritischen Bedingungen des Drucks und der Temperatur in der fluiden Phase ohne Qualitätsverlust der Bierhefe entfernt.

2. Verfahren nach Anspruch 1, dadurch gekennzeichnet, daß man bei Drucken von solchen über dem kritischen Druck bis 295 bar, vorzugsweise von 98 bis 200 bar arbeitet.

3. Verfahren nach Ansprüchen 1 und 2, dadurch gekennzeichnet, daß man bei Temperaturen von solchen über der kritischen Temperatur bis 100°C, vorzugsweise 40 bis 70°C arbeitet.

4. Verfahren nach Ansprüchen 1—3, dadurch gekennzeichnet, daß die gebrauchte Bierhefe mit dem überkritischen $CO_2$ im Gleichstrom behandelt wird.

5. Verfahren nach Ansprüchen 1—3, dadurch gekennzeichnet, daß die Behandlung zusätzlich im stationären Zustand durchgeführt wird.

6. Verfahren nach Ansprüchen 1 bis 5, dadurch gekennzeichnet, daß das mit Bitterstoffen beladene Gas durch ein Adsorptionsmittel zur Entfernung der Bitterstoffe geleitet und anschließend im Kreislaufverfahren wiederverwendet wird.

### Revendications

1. Procédé d'élimination des substances amères de la levure de bière épuisée dans des systèmes aqueux, caractérisé en ce qu'on élimine les substances amères en traitant une suspension aqueuse et/ou une solution colloïdale aqueuse de la levure de bière épuisée ou de la levure de bière séchée, par $CO_2$, de l'éthane, de l'éthylène et/ou du propane dans des conditions supercritiques de pression et de température en phase fluide, sans perte de qualité de la levure de bière.

2. Procédé selon la revendication 1, caractérisé en ce qu'on travaille à des pressions supérieures à la pression critiques jusqu'à 295 bars, de préférence entre 98 et 200 bars.

3. Procédé selon les revendications 1 et 2, caractérisé en ce qu'on travaille à des températures supérieures à la température critique jusqu'à 100°C, de préférence entre 40 et 70°C.

4. Procédé selon les revendications 1 à 3, caractérisé en ce qu'on soumet la levure de bière épuisée à un traitement par du $CO_2$ supercritique s'écoulant dans le même sens.

5. Procédé selon les revendications 1 à 3, caractérisé en ce qu'on effectue le traitement en outre à l'état stationaire.

6. Procédé selon les revendications 1 à 5, caractérisé en ce qu'on fait passer le gaz, chargé des substances amères, à travers un agent d'absorption pour éliminer les substances amères et l'on utilise à nouveau le gaz que l'on recycle.

### Claims

1. A process for the removal of the bitter substances from spent brewer's yeast in aqueous systems, characterized in that the bitter substances are removed by treating an aqueous suspension and/or a colloidal aqueous solution of the spent brewer's yeast or of dried brewer's yeast with $CO_2$, ethane, ethylene and/or propane under hypercritical conditions of pressure and temperature in the fluid phase without loss in quality of the brewer's yeast.

2. The process according to claim 1, characterized in that the work is carried out at pressures above the critical pressure up to 295 bar, preferably from 98 to 200 bar.

3. The process according to claims 1 and 2, characterized in that the work is carried out at temperatures above the critical temperature up to 100°C, preferably from 40°C to 70°C.

4. The process according to claims 1 to 3, characterized in that the spent brewer's yeast is treated with the hypercritical $CO_2$ flowing in the same direction.

5. The process according to claims 1 to 3, characterized in that the treatment in addition is carried out in a stationary state.

6. The process according to claims 1 to 5, characterized in that the gas loaded with the bitter substances is passed through an adsorbing agent for the removal of the bitter substances and is then reused in the circulating process.